# EUROPEAN PATENT SPECIFICATION

(11) **EP 1 742 638 B1**
(45) Date of publication and mention of the grant of the patent: **14.12.2011**
(21) Application number: 05738246.7
(22) Date of filing: 25.04.2005
(51) Int. Cl.: A61K 31/549, A61K 31/63, A61K 31/64, A61K 31/704, A61K 45/06, A61K 47/10, A61P 27/02

(54) **COMPOSITION COMPRISING A DIURETIC AND CARDIAC GLYCOSIDE FOR THE TREATMENT OF DNA VIRAL INFECTIONS OF THE EYE**
ZUSAMMENSETZUNG MIT EINEM DIURETIKUM UND HERZGLYKOSID ZUR BEHANDLUNG VON DNA-VIRUSINFEKTIONEN DES AUGES
COMPOSITION COMPORTANT UN DIURETIQUE ET UN GLYCOSIDE CARDIAQUE POUR LE TRAITEMENT DES INFECTIONS A VIRUS A ADN DE L'OEIL

(30) Priority: 23.04.2004 GB 0409102
(43) Date of publication of application: 17.01.2007
(73) Proprietor: Henderson Morley Plc, Moseley, Birmingham B13 8JS (GB)
(72) Inventor: HARTLEY, Christopher Edward, Henderson Morley PLC, Moseley, Birmingham (GB)
(74) Representative: Moore, Christopher Mark
(86) International application number: PCT/GB2005/001570
(87) International publication number: WO 2005/102351

(56) References cited:
- WO-A-01/49242
- US-A- 5 494 901
- "Rote Liste 2002 - Arzneimittelverzeichnis für Deutschland", 1 January 2002 (2002-01-01), Rote Liste Service GmbH - Editio Cantor Verlag, Frankfurt/Main * page 67001 * * 67003. Digophton Augentropfen *
- Datasheet Digofton (Digophton)

## Description

The invention relates to prophylactic and therapeutic treatment of DNA viral infections specifically to DNA viral infections of the eye.

DNA viruses have a central core of DNA within a proteinaceous structure. The DNA carries the genetic code to reproduce the virus. Viruses must infect a living cell to reproduce and, once inside the cell, the replication of the viral DNA is essential for virus infectivity. There are numerous viral proteins that are well characterised including important enzymes which act as ideal targets for antiviral chemotherapy. These include DNA polymerase and thymidine kinase which are needed for DNA replication.

We have established that a loop diuretic and a cardiac glycoside or a lithium salt act synergistically in the treatment of DNA viral infections. Thus, in international patent application PCT/GB00/04790 (WO 01/49300) we have described and claimed use of a loop diuretic and lithium to exert a synergistic effect in the treatment of DNA viral infections, and compositions useful for the treatment of DNA viral infections comprising a synergistic combination of a loop diuretic and lithium. In international patent application PCT/GB00/04793 (WO 01/49242) we have described and claimed use of a loop diuretic and a cardiac glycoside to exert a synergistic effect in the treatment of DNA viral infections, together with compositions useful in the treatment of DNA virus infections comprising a synergistic combination of a loop diuretic and a cardiac glycoside. Both of these documents describe *in vitro* experiments which establish the efficacy of the treatment.

The cardiac glycosides may be any one or more of digoxin, digitoxin, medigoxin, lanatoside C, proscillaridin, k strophanthin, peruvoside and ouabain. Plants of the digitalis species (e.g. digitalis purpura, digitalis lanata) contain cardiac glycosides such as digoxin and digitoxin which are known collectively as digitalis. Other plants contain cardiac glycosides which are chemically related to the digitalis glycosides and these are often also referred to as digitalis. Thus the term digitalis is used to designate the whole group of glycosides; the glycosides are composed of two components a sugar and a cardenolide. Ouabain is derived from an African plant Strophanthus gratus (also known as strophanthidin G) and can be available in intravenous form (it is not absorbed orally) and is used for many laboratory experiments in the study of glycosides, because of its greater solubility. It has a virtually identical mode of action as digoxin.

Digoxin is described chemically as (3b, 5b, 12b)-3-[*0*-2,6-dideoxy-b-*D-riob*-hexopyranosyl-(1"4)-*O*-2,6-dideoxy-b-*D-ribo*-hexopyranosyl-(1"4)-2,6-dideoxy-b-*D-ribo*-hexopyranosyl) oxy]-12,14-dihydroxy-card-20-22)-enolide. It has a molecular formula of C₄₁H₆₄O₁₄, and a molecular weight of 780.95. Dixogin exists as odourless white crystals that melt with decomposition above 230°C. The drug is practically insoluble in water and in ether; slightly soluble in diluted (50%) alcohol and in chloroform; and freely soluble in pyridine.

Frusemide, also known as furosemide, is an anthrilic acid derivative, chemically 4-chloro-N-furfuryl-5-sulfamoylanthranilic acid. It is practically insoluble in water at neutral pH, however is freely soluble in alkali. Furosemide exerts its physiological effect by inhibition of the transport of chloride ions across cell members. Furosemide is a loop diuretic with a short duration of action. It is used for treating oedema due to hepatic, renal, or cardiac failure and treating hypertension. The bioavailability of furosemide is between 60% to 70% and it is primarily excreted by filtration and secretion as unchanged drug. Furosemide acts on the Na+/K+/2CI- cotransporter. For its diuretic effect, its predominant action is in the ascending limb of the loop of Henlé in the kidney. Loop diuretics markedly promote K⁺ excretion, leaving cells depleted in intracellular potassium. This may lead to the most significant complication of long term systemic furosemide usage, namely a lowered serum potassium. We postulate that it is this action which makes loop diuretics useful as an agent against DNA viruses.

Evidence suggests that the major biotransformation product of furosemide is a glucuronide Furosemide is extensively bound to plasma proteins, mainly albumin. Plasma concentrations ranging from 1 to 400 µg/ml are 91-99% bound in healthy individuals. The unbound fraction ranges between 2.3-4.1 % at therapeutic concentrations. The terminal half life of frusemide is approximately 2 hours, and it is predominantly excreted in the urine.

The compositions of WO 01/49242 may be adapted for external or internal administration. Topical and systemic applications are said to be likely to be the most useful. It is stated that a much preferred feature of the invention that the compositions are formulated for topical application. Other ingredients may be present, provided that they do not compromise the anti-viral activity; an example is a preservative.

In our previous application we stated that preferred treatment mode of the invention was the use of local concentrations of loop diuretic and cardiac glycoside for the highly effective treatment of virus infections of the eye. Recurrent Herpes infections of the cornea in man is the most common viral cause of blindness.

We stated that contact lenses carrying a loop diuretic and a cardiac glycoside (*e.g*. impregnated therewith) would be a safe and efficient method for creating high intracellular concentrations to prevent or treat the disease. We also stated that a depot application of a loop diuretic and cardiac glycoside applied intra-occularly would be a suitable method for the treatment of cytomegalovirus retinitis, a major cause of blindness in patients suffering AIDS. It was further stated that the invention provided a combination of frusemide and digoxin as a topical application in a buffered saline formulation for the treatment of corneal eye infections.

However, to date it has been impossible to formulate a medicament which can be applied topically to or around the eye, due to the solubility characteristics of loop diuretics and furosemide and the sensitivity of the eye.

As is well known, the eye is a very sensitive organ which will only tolerate low levels of pollutants. If the normal response mechanisms (tear generation and/or blinking *etc*.) are not sufficient to remove the pollutant then substantial damage can occur.

One substance to which the eye is particularly sensitive is ethanol. Ethanol solutions are used to debride the corneal epithelium before photorefractive keratectomy and laser subthelial keratectomy (LASEK). It has also been established that ethanol has a direct toxic effect on stromal proteins (see Gruters et al in Opthalmologe; 2002; 99(4), 266-269). Accordingly, the use of concentrated ethanolic solutions for topical treatments of the eye is discouraged.

It is one object of this invention to provide a preparation for the treatment of a DNA viral infection of the eye which may be applied topically or as a depot and which contains sufficient active ingredients to treat or prevent DNA viral infections.

It has been surprisingly found that a preparation of a synergistic amount of a diuretic and a cardiac glycoside may be formulated in an ethanolic solution for use in the eye.

Accordingly, in a first aspect of the invention there is provided a method of making a composition for the treatment or prevention of DNA viral infection of the eye, the method comprising dissolving a diuretic and a cardiac glycoside in a solvent comprising ethanol and diluting the so-formed solution with water to provide a composition containing a content of ethanol which is acceptable for use in or about the eye comprising diluting the so-formed solution with water to reduce the ethanol content to about 2 to about 2.5 v/v %. The diuretic and cardiac glycoside may be dissolved in about 60 to about 95 v/v % solution of ethanol in water.

Most preferably, the diuretic and cardiac glycoside are dissolved in a 90 v/v % solution of ethanol in water.

Sufficient water is added to reduce the ethanol content to about 2 to about 2.5 v/v % in the finished preparation.

A second aspect of the invention provides a composition for the treatment or prevention of DNA viral infection of the eye, the composition comprising a synergistic amount of a diuretic and a cardiac glycoside dissolved in a water/ethanol solution wherein the content of ethanol is adjusted so that the composition is suitable for use in or about the eye and the ethanol content is about 2 to about 2.5 v/v %. Where water is referred to above, it will be appreciated that further substances may be dissolved in the water, the water may be a saline, for example a buffered saline, solution for example.

The so-formed solution of cardiac glycoside and diuretic in ethanol/water may be combined with further substances to form, say, a lotion or cream for use in or around the eye, or it may be used as eye drops. Preservatives and/or stabilisers may be used also.

In a further aspect of the invention the diuretic or cardiac glycoside may be dissolved in ethanol and used in isolation to form a composition for the treatment of DNA viral infections of the eye in which only one active species is present.

In the specification, the terms 'eye-acceptable' and 'acceptable to the eye' (as well as similar terms) are intended to mean that no lachrymatory effect is seen and/or no irritation of, and/or damage to, the surface of the eye (cornea) and/or the surrounds of the eye (the lids, tear ducts *etc*.) and/or the deeper structures within the eye (lens, muscles *etc*.).

The amount of the or each active ingredient used will be determined according to whether the DNA viral infection, or the sequelae thereof, is to be treated or if the composition is to be used as a preventative measure. In either or all cases, the dosage will be determined empirically in each case by the technician using the normal standards of skill in the art. Regard will also be had, when more than one active is present, to adjust the proportion of the ingredients to exert a synergistic effect.

The diuretic may be selected from a range of loop diuretics, such as sulphonamides and thiazides. We include in this context sulphonylureas.

Loop diuretics are substances which act on the ascending loop of Henlé in the kidney. They are sulphonamides but may be other substances too. Typical examples include:

| | |
|---|---|
| acetazolamide | mefruside |
| ambuside | methazolamide |
| azosemide | piretanide |
| bumetanide | torsemide |
| butazolamide | tripamide |
| chloraminophenamide | xipamide |
| clofenamide | |
| clopamide | ethacrynic acid |
| clorexolone | etozolin |
| disulfamide | ticrynafen |
| ethoxzolamide | |
| furosemide (frusemide) | |

Thiazide diuretics include the benzothiadriazines derivatives, also known as thiazides. Typical examples are:

| | |
|---|---|
| althiazide | hydrobenzthiazide |
| bemetizide | hydrochlorothiazide |
| bendroflumethiazide | hydrofluoromethiazide |
| benzthiazide | indapamide |
| benzylhydrochlorothiazide | mebutizide |
| buthiazide | methylcyclothiazide |
| chlorothiazide | meticane |
| chlorothalidone | metalazone |
| cyclopenthiazide | paraflutizide |
| cyclothiazide | polythiazide |
| epithiazide | quinethazone |
| ethiazide | teclothiazide |
| fenquizone | trichlormethiazide |

Sulphonylureas are anti-diabetic drugs which influence ion transport across cell membranes. They are instanced by:

| | |
|---|---|
| acetohexamide | glyburide |
| 1-butyl-3-metanilylurea | glybuthiazole |
| carbutamide | glybuzole |
| chlorpropamide | glycycloamide |
| glibenclamide | glyclopyramide |
| glibornuride | glyhexamide |
| gliclazide | glymidine |
| glimepiride | glypinamide |
| glipizide | phenbutamide |
| gliquidone | tolazamide |
| glisentide | tolbutamide |
| glisolamide | tolcylamide |
| glisoxepid | |

Preferably the diuretic is loop diuretic and is any one or more of frusemide, bumetanide, ethacrynic acid or torasemide. According to our studies loop diuretics mediate their antiviral effects through alteration to the cellular concentration of ions, cellular ionic balances, cellular ionic milieu and electrical potentials.

Cardiac glycosides exhibit positive inotropic activity which is mediated by inhibition of sodium-potassium adenosine triphosphate (Na/K-ATPase). Substances which fall into this category include:

| | |
|---|---|
| acetyldigitoxon | gitalin |
| acetyldigoxin | gitoxin |
| cymarin | Lanatoside C |
| deslanoside | medigoxin |
| Digitalin | meproscillarin |
| Digitalis Lanata leaf | oubain |
| Digitalis Leaf | peruvoside |
| Digitoxin | proscillaridin |
| Digoxin | strophanthin-K |

Preferably the cardiac glycosides may be any one or more of digoxin, digitoxin, and ouabain.

As stated in our earlier patent application (WO 01/49242), at the cellular level digitalis exerts its main effect by the inhibition of the sodium transport enzyme sodium potassium adenosine triphosphatase (Na/K ATPase); this is directly responsible for the electrophysiological effects of heart muscle and according to our understanding also activity against DNA viruses. This activity also has an effect on the efficiency of myocardial contractility due to secondary changes in intracellular calcium. At very low intracellular concentrations of digitalis, the opposite effects can be seen with a reduced efficiency of cardiac contractions as the digitalis stimulates the Na/K ATPase.

A preferred combination is the loop diuretic frusemide and the cardiac glycoside digoxin. One preferred concentration is frusemide 7mg/mℓ and digoxin 62µg/mℓ. It is within the scope of the invention to separate the application of the two active ingredients by a short time period.

Studies (including X-ray microanalysis) have demonstrated the anti-viral DNA effects of a composition of the invention are dependent on a depletion of intracellular potassium ions. Briefly these studies demonstrate:
- replacement of potassium will restore DNA synthesis;
- use of frusemide and digoxin in combination have comparable effects to potassium depletion;
- the level of potassium depletion is sufficient to allow normal cell function;
- the potassium depletion has no cytotoxic effects.

Thus, by altering the cellular concentrations of ions, cellular ionic balances, cellular ionic milieu and cellular electrical potentials by the application of a loop diuretic and a cardiac glycoside it is possible to change the metabolism of the cell without detriment to the cell but so that virus replication is inhibited. Accordingly, we are confirmed in the view that the use of a loop diuretic and a cardiac glycoside is of benefit in preventing or controlling virus replication by inhibiting the replication of viral DNA.

Application of a loop diuretic and cardiac glycoside in an ethanol solution applied intra-occularly is a suitable method for the treatment of cytomegalovirus (CMV) retinitis as well as other occcular DNA viral infections.

Application of an eye drop comprising loop diuretic and cardiac glycoside dissolved in ethanol is a suitable method for the treatment of adenoviral keratoconjunctivitus.

Some other viral infections which may be treated are herpes simplex type 1 and 2 and varicella zosler virus, all of which are able to infect epithelial cells and cause sight-threatening disease.

The invention is intended for general human or veterinary use and is thus of value in treating DNA viral infections in man or animals, e.g. cats, dogs; pigs, cattle, sheep, horses; poultry; fish; wild animals; and the like.

It has been found that 2.5 v/v % of ethanol is the maximum concentration which is acceptable in and to the eye.

In an evaluation, single dose units of 0.4mℓ having a strength of 62.5µg/mℓ digoxin and 7.5mg/mℓ frusemide were made of the following formulation:

**Table 1. The formulation**

| | |
|---|---|
| digoxin | 0.025mg |
| frusemide | 3mg |
| ethanol (96%) | 10mg |
| propylene glycol | 40mg |
| NaCl | 2.248mg |
| citric acid | 0.0375mg |
| disodium ammonium hydrogen phosphate | 0.094mg |
| NaOH(1N) | 9.96mg |
| water | ad400mg |

In order to assess the effectiveness of the treatment against adenoviral keratoconjunctivitus, a double blind experiment was conducted with 12 patients.

Trial medication was administered as eye drops at Visit 1. An eye drop made to the above formulation was administered into a randomly chosen eye in a double-blind manner and placebo was administered into the contralateral eye. The dosage was four drops (one drop contained 26 µℓ) into each eye every two hours over a period of 10 hours. Altogether there were six applications of the formulation and placebo always into the same eyes. The trial medication has to be instilled successively into both eyes; there has to be instilled 4 drops of the vial "R" into the right eye and 4 drops of the vial "L" into the left eye.

After six applications, the dose to be administered in this trial was projected to be 39 µg digoxin and 4.68 mg furosemide per day. On a mg per kg basis, the projected dose to be tested in humans equated to 0.78 µg/kg digoxin and 93.6 µg/kg furosemide (assuming a 50 kg individual) and to 0.56 µg/kg digoxin and 66.9 µg/kg furosemide (assuming a 70 kg individual).

The dosage in this trial was calculated from the following oral dosage form in humans:

### Digoxin:

Calculated from an oral dosage form: bioavailability is about 80%; average therapeutic plasma concentration = 1,5 µg/ℓ; daily reduction quote is 20% and the half-life is 42 hours.

In order to reach a therapeutic plasma level by oral dosage, an initial dose of 0.5 - 0.75 mg per day is given over three to five days. To keep this concentration, a maintenance dose of 0.25 - 0.375 mg per day is given. Keeping the bioavailability in mind, the initial dose is 0.4 - 0.6 mg in the body for the first three to five days and the maintenance dose is 0.2 - 0.3 mg per day in the body.

Within the formulation the concentration of digoxin was 62.5 µg/mℓ. By calculating 4 x 26 µℓ = 104 µℓ drops in one eye, a dose of 6.5 µg was reached per application in one eye. After 6 applications per day, a daily dose of 39 µg was reached.

This was 10 times lower than the initial dose (400 µg) and 5 times lower than the maintenance dose (200 µg) for oral dosage.

### Furosemide:

Calculated from an oral dosage form: bioavailability is 70%; normal dose is 20 - 40 mg per day in order to reach a serum level of 14 - 28 mg per day.

Within the formulation, the concentration of furosemide was 7.5 mg/mℓ. By calculating 4 x 26 µℓ = 104 µℓ drops in one eye, a dose of 0.78 mg was reached per application in one eye. After 6 applications per day, a daily dose of 4.68 mg was reached.

This was three times lower than the daily therapeutic dose (14 mg) calculated for the serum after oral dosing.

The eye (right or left) for each subject's active treatment was determined through the assignment of an unique random number in a double-blind manner.

After Screening, eligible subjects proceeded into a one-day treatment period (Visit 1) with six administrations of trial medication once every two hours over a period of 10 hours. All twelve subjects received four drops of the formulation in a randomly chosen eye and four drops of placebo in the contralateral eye. Before each administration of trial medication, assessment of vital signs and eye examinations were performed. Additional examinations were performed 11.75, 24, 36, 48 and 72 hours after the first administration of trial medication.

All assessments for each subject were performed, as far as possible, at the same time of the day.

### Visit 1

Visit 1 took place within two weeks after the Screening/Enrolment Visit.

For a period of ten hours, every two hours four drops of the formulation were instilled into a randomly chosen eye and placebo were instilled into the contralateral eye. Altogether there were six instillations of the fomulation and placebo.

Before the first instillation, subjects were assessed as follows:
- Adverse Events
- Concomitant Medication
- Vital signs (blood pressure, pulse rate)
- Visual acuity
- Intraocular pressure
- Ophthalmoscopy
- Slitlamp examination
- Corneal fluorescein staining
- Blood sampling for digoxin and potassium levels
- Randomisation

1.75 hours after each instillation, subjects were assessed as follows:
- Adverse Events
- Vital signs (blood pressure, pulse rate)
- Visual acuity
- Intraocular pressure
- Ophthalmoscopy
- Slitlamp examination
- Corneal fluorescein staining

1.75 hours after the last instillation, subjects were assessed as follows:
- Adverse Events
- Vital signs (blood pressure, pulse rate)
- Visual acuity
- Intraocular pressure
- Ophthalmoscopy
- Slitlamp examination
- Corneal fluorescein staining
- Blood sampling for digoxin and potassium levels

### Visit 2

Visit 2 took place 24 hours after the first instillation of trial medication. At Visit 2, subjects were assessed as after the first installation.

### Visit 3

Visit 3 took place 36 hours after the first instillation of trial medication. At Visit 3, subjects were assessed as per Visit 2.

### Visit 4

Visit 4 took place 48 hours after the first instillation of trial medication. At Visit 4, subjects were assessed as per Visit 2.

### Visit 5 Termination Visit)

Visit 5 took place 72 hours after the first instillation of trial medication. At Visit 5, subjects were assessed as follows:
- Adverse Events
- Concomitant Medication
- Vital signs (blood pressure, pulse rate)
- Visual acuity
- Intraocular pressure
- Ophthalmoscopy
- Slitlamp examination
- Corneal fluorescein staining
- Blood sampling for safety laboratory test
- Blood sampling for digoxin and potassium levels
- Urinalysis
- Physical examination
- ECG
- Questionnaire about ocular symptoms

### Safety Follow-Up Visit

The Safety Follow-up Visit took place seven to fourteen days after Visit 5, in case of unresolved adverse events at the end of the trial (Visit 5). At the Safety Follow-up Visit, subjects were assessed as follows:
- Adverse Events
- Concomitant Medication
- Vital signs (blood pressure, pulse rate)
- Visual acuity
- Intraocular pressure
- Ophthalmoscopy
- Slitlamp examination
- Corneal fluorescein staining

In all cases, the characteristic keratitis reduced over the period of treatment and all infection had disappeared within three weeks.

The formulation can also be presented as a so-called Vitrasert implant which is surgically placed in the eye for the treatment of CMV retinitis to allow the formulation to combat the symptoms of CMV retinitis.

The diuretic can be altered for any of the loop diuretics, thiazides or sulphonylureas. The cardiac glycoside can be chosen from any of the above-disclosed group.

In all cases the formulation of cardiac glycoside and/or diuretic in an ethanolic solution was found to be acceptable to the eye.

The composition of the invention may also be useful for the treatment of DNA viral infections outside of the eye.

## Claims

1. A method of making a composition for the treatment or prevention of DNA viral infection of the eye, the method comprising dissolving a diuretic and a cardiac glycoside in a solvent comprising ethanol and diluting the so-formed solution with water to provide a composition containing a content of ethanol which is acceptable for use in or about the eye comprising diluting the so formed solution with water to reduce the ethanol content to about 2 to about 2.5 v/v %.

2. A method according to Claim 1, comprising dissolving the diuretic and the cardiac glycoside in a solvent comprising about 60 to about 95 v/v % solution of ethanol in water.

3. A method according to Claim 2, wherein the solvent comprises a 90 v/v % solution of ethanol in water.

4. A method according to any preceding Claim, wherein the diuretic is one or more of a loop diuretic, a sulphonamide or thiazide.

5. A composition for the treatment or prevention of DNA viral infection of the eye, the composition comprising a diuretic and cardiac glycoside in synergistic proportion, dissolved in a water/ethanol solution wherein the content of ethanol is adjusted so that the composition is suitable for use in or about the eye and the ethanol content is about 2 to about 2.5 v/v %.

6. A composition for the treatment or prevention of DNA viral infection of the eye, the composition comprising a cardiac glycoside and a diuretic dissolved in a water/ethanol solution wherein the content of ethanol is adjusted so that the composition is suitable for use in or about the eye and the ethanol content is about 2 to about 2.5 v/v %.

7. A composition according to Claim 5 or 6, wherein the composition comprises eye drops or is presented as a lotion or cream.

8. A composition according to Claim 5 to 7, wherein the diuretic is one or more of a loop diuretic, sulphonylurea or thiazide.

9. A composition according to any of Claims 5 to 8, wherein the cardiac glycoside is digoxin, digitoxin, ouabain or strophanthin.

## Patentansprüche

1. Ein Verfahren zur Herstellung einer Zusammensetzung zur Behandlung oder Prävention einer DNA-Virusinfektion des Auges, wobei das Verfahren das Lösen eines Diuretikums und eines Herzglykosids in einem Lösungsmittel, welches Ethanol umfasst, und das Verdünnen der so gebildeten Lösung mit Wasser umfasst, um eine Zusammensetzung bereit zu stellen, welche einen Ethanolgehalt enthält, der für die Verwendung im oder am Auge akzeptabel ist, was das Verdünnen der so gebildeten Lösung mit Wasser umfasst, um den Ethanolgehalt auf ca. 2 bis ca. 2,5 Vol.-% (v/v) zu verringern.

2. Ein Verfahren gemäß Anspruch 1, welches das Lösen des Diuretikums und des Herzglykosids in einem Lösungsmittel umfasst, welches eine ca. 60 bis ca. 95 Vol.-% Lösung von Ethanol in Wasser umfasst.

3. Ein Verfahren gemäß Anspruch 2, wobei das Lösungsmittel eine 90 Vol.-% Lösung von Ethanol in Wasser umfasst.

4. Ein Verfahren gemäß irgendeinem vorhergehenden Anspruch, wobei das Diuretikum eines oder mehrere aus einem Schleifendiuretikum, einem Sulfonamid oder Thiazid ist.

5. Eine Zusammensetzung zur Behandlung oder Prävention einer DNA-Virusinfektion des Auges, wobei die Zusammensetzung ein Diuretikum und ein Herzglykosid in synergistischem Verhältnis umfasst, welche in einer Wasser/Ethanol-Lösung gelöst sind, wobei der Gehalt an Ethanol so eingestellt ist, dass die Zusammensetzung zur Verwendung im oder am Auge geeignet ist und der Ethanolgehalt ca. 2 bis ca. 2,5 Vol.-% beträgt.

6. Eine Zusammensetzung zur Behandlung oder Prävention einer DNA-Virusinfektion des Auges, wobei die Zusammensetzung ein Herzglykosid und ein Diuretikum umfasst, welche in einer Wasser/Ethanol-Lösung gelöst sind, wobei der Gehalt an Ethanol so eingestellt ist, dass die Zusammensetzung zur Verwendung im oder am Auge geeignet ist und der Ethanolgehalt ca. 2 bis ca. 2,5 Vol.-% beträgt.

7. Eine Zusammensetzung gemäß Anspruch 5 oder 6, wobei die Zusammensetzung Augentropen umfasst oder als eine Lotion oder Creme vorliegt.

8. Eine Zusammensetzung gemäß Anspruch 5 bis 7, wobei das Diuretikum eines oder mehrere aus einem Schleifendiuretikum, Sulfonylharnstoff oder Thiazid ist.

9. Eine Zusammensetzung gemäß irgendeinem der Ansprüche 5 bis 8, wobei das Herzglykosid Digoxin, Digitoxin, Ouabain oder Strophanthin ist.

## Revendications

1. Procédé pour fabriquer une composition destinée au traitement ou à la prévention d'une infection de l'oeil par un virus à ADN, le procédé consistant à dissoudre un diurétique et un glycoside cardiaque dans un solvant comprenant de l'éthanol et à diluer la solution ainsi formée avec de l'eau afin d'obtenir une composition contenant une teneur en éthanol qui est acceptable pour une utilisation dans ou autour de l'oeil, qui consiste à diluer la solution ainsi formée avec de l'eau afin de réduire la teneur en éthanol à environ 2 à 2,5 % v/v.

2. Procédé selon la revendication 1, qui consiste à dissoudre le diurétique et le glycoside cardiaque dans un solvant comprenant une solution d'éthanol à environ 60 à 95 % v/v dans de l'eau.

3. Procédé selon la revendication 2, dans lequel le solvant comprend une solution d'éthanol à 90 % v/v dans de l'eau.

4. Procédé selon l'une quelconque des revendications précédentes, dans lequel le diurétique est l'un ou plusieurs parmi un diurétique de l'anse, un sulfamide ou un thiazidique.

5. Composition destinée au traitement ou à la prévention d'une infection de l'oeil par un virus à ADN, la composition comprenant un diurétique et un glycoside cardiaque en proportion synergistique, dissous dans une solution eau/éthanol dans laquelle la teneur en éthanol est ajustée de sorte que la composition soit appropriée pour une utilisation dans ou autour de l'oeil et que la teneur en éthanol soit d'environ 2 à environ 2,5 % v/v.

6. Composition destinée au traitement ou à la prévention d'une infection de l'oeil par un virus à ADN, la composition comprenant un glycoside cardiaque et un diurétique dissous dans une solution eau/éthanol dans laquelle la teneur en éthanol est ajustée de sorte que la composition soit appropriée pour une utilisation dans ou autour de l'oeil et que la teneur en éthanol soit d'environ 2 à environ 2,5 % v/v.

7. Composition selon la revendication 5 ou 6, où la composition comprend des gouttes oculaires ou est présentée sous forme de lotion ou de crème.

8. Composition selon la revendication 5 à 7, dans laquelle le diurétique est l'un ou plusieurs parmi un diurétique de l'anse, une sulfonylurée ou un thiazidique.

9. Composition selon l'une quelconque des revendications 5 à 8, dans laquelle le glycoside cardiaque est la digoxine, la digitoxine, l'ouabaïne ou la strophantine.
